# EUROPEAN PATENT SPECIFICATION

(11) **EP 1 581 120 B1**
(45) Date of publication and mention of the grant of the patent: **19.08.2009**
(21) Application number: 03770843.5
(22) Date of filing: 24.11.2003
(51) Int. Cl.: A61B 17/00, A61B 17/12

(54) **AN IMPLANT FOR OCCLUDING A PASSAGE**
IMPLANTAT ZUM VERSCHLIESSEN EINER KÖRPERÖFFNUNG
IMPLANT PERMETTANT L'OCCLUSION D'UN PASSAGE

(30) Priority: 27.11.2002 US 306481
(43) Date of publication of application: 05.10.2005
(73) Proprietor: Carag AG, 6340 Baar (CH)
(72) Inventor: SOLYMAR, Laszlo, S-427 36 Billdal (SE); THOMMEN, Daniel, Thomas, CH-6312 Steinhausen (CH); BERNHARD, Jerome, CH-8001 Zürich (CH)
(74) Representative: Bremi, Tobias Hans
(86) International application number: PCT/CH2003/000776
(87) International publication number: WO 2004/047649

(56) References cited:
- EP-A- 0 927 540
- WO-A-02/38051
- US-B1- 6 270 515
- US-B1- 6 355 052
- US-B1- 6 432 134

## Description

### FIELD OF THE INVENTION

The present invention generally relates to an implant device for closing a body passage (e.g., an aperture through the atrial diaphragm or septum or the ventricle diaphragm or septum of a heart) or in a body channel, and more particularly comprising an occluding or closing body which is expanded and fixed into place at the passage area to be occluded/closed.

### BACKGROUND OF THE INVENTION

By way of example EP-A-0 362 113 shows a device for closing a passage in the heart of patients, wherein the closing part runs the risk of tipping over and thereby causing the passage to be exposed in passing through the heart. The cause of the non-secure closing capacity is the relative unfixed maneuverability for the closing part when applied around the passage, and that the application takes part long before it has arrived in its final position around the passage in the heart.

WO 02/38051 discloses implants for occluding a passage in a circulatory system. This implant has its particular application as a cardiological implant by means of which it is possible, for example, to close an aperture through the auricle diaphragm or the ventricle diaphragm of a heart. It is arranged to be deployed or built up (i.e., assembled) at a desired location in the body (e.g., the heart), in contrast to known other implants (e.g., so-called umbrellas and sails) that are instead extended as soon as the compressed umbrella leaves its insertion sheath. It includes a plurality of thin wire-like members each having a proximal and a distal end. The members are made of non-bendable material. Two holders are provided to which these ends are attached. When the distance between the two holders is reduced the non-bendable members are caused to execute a twisting motion yielding in a plurality of radially extending loops, which are fixed in their position. In one embodiment, a balloon structure is expanded. In another embodiment, an occluding body arranged in the middle between the two holders is expanded by the twisting members. WO 02/38051 suggests to use nitinol as material for the wire-like members or threads.

The aim of a stent according to US 6,432,134 Bl is to provide a short device that is adapted to close also openings that are not stretched very much in the longitudinal dimension. The stent for occluding the human doctus arteriosus comprises a wire of shape memory effect or superelastic material. The wires are expandable from a relatively straightened state for introduction into the patient, to an occluding. According to one embodiments the material chosen for the wires are shape memory effect materials. Their elastic recoil will ensure that the implant has adapted the minimum length possible within the anatomy and that it therefore projects as little as possible into the vessel on either side of the implant.

It is therefore an object of the present invention to provide, based on the device disclosed in WO 02/38051, an occlusive device being more acceptable by a living body, especially by a human body.

### SUMMARY OF THE INVENTION

This object is achieved by means of a device according to the present invention, which is substantially characterized in that it comprises
a plurality of thin, preferably stiff, members each having a proximal and a distal end;
a first holder to which the distal ends of said members are attached;
a second holder to which the proximal ends of said members are attached;
an expansible, preferably foldable, occluding body attached to said members;
said plurality of members with said occluding body forming an elongated article extending along a longitudinal axis and being adapted for insertion through a delivery mechanism in an insertion condition;
said members being attached to said first and second holders and the distance between said holders being reduceable in a manner to cause said members to execute a twisting motion relative to said axis to yield a plurality of generally radially extending loops forming at least one fixation structure, thereby expanding, preferably unfolding, the occluding body and
said at least one fixation structure being fixable in a final occluding condition.

The plurality of members are formed completely of biodegradable material, which consists of polydioxanone, polycaprolactone or polygluconate comprising a tensile strength of from about 103,42 N/mm² to about 413,69 N/mm² (15 thousands of ponds per square inch (ksi) to about 60 ksi) and / or comprising a tensile modulus of from about 1378,95 N/mm² to about 4826,33 N/mm² 200,000 pounds per square inch (psi) to about 700,000 psi).

The implant according to the invention is formed partly or entirely of biodegradable or bioabsorbable material(s). A living body can degrade or absorb it, therefore minimizing the risk of non-compliance with the immunological system of the living body.

Usually, an intravenous delivery mechanism is used. However other delivery mechanisms, such as one using the esophagus can be used.

In a preferred embodiment, the occluding body is made of biodegradable or bioabsorbable material.

These and other advantages and objects achieved by the present invention will be further appreciated and understood upon consideration of the following detailed description of certain embodiments taken in conjunction with the drawings in which:

### BRIEF DESCRIPTION OF THE DRAWINGS

- FIG. 1: shows an initial position for an implant, the implant being seen from the side and from one end respectively;
- FIG. 2: shows the implant of FIG. 1 at a between position during extension seen from the side and one end respectively;
- FIG. 3: shows the implant of FIG. 1 completely extended seen from the side and one end respectively;

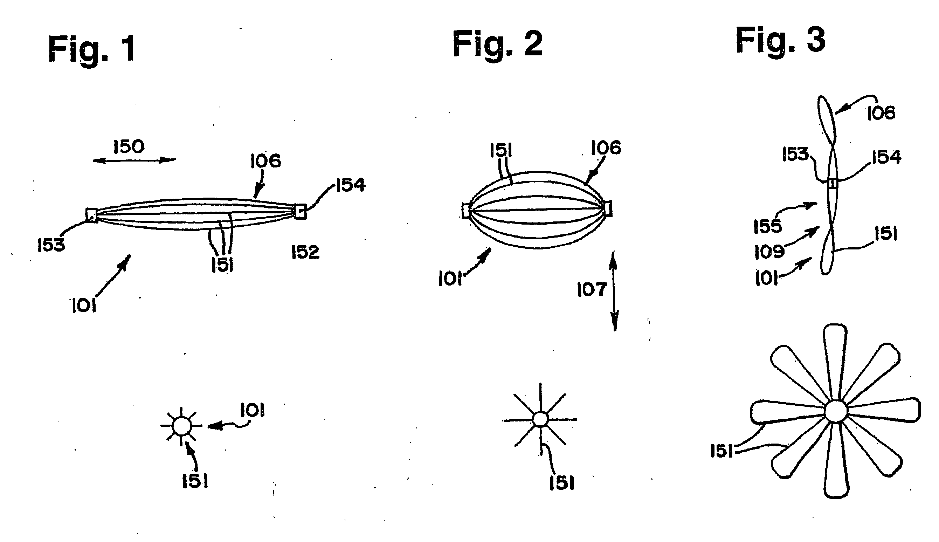

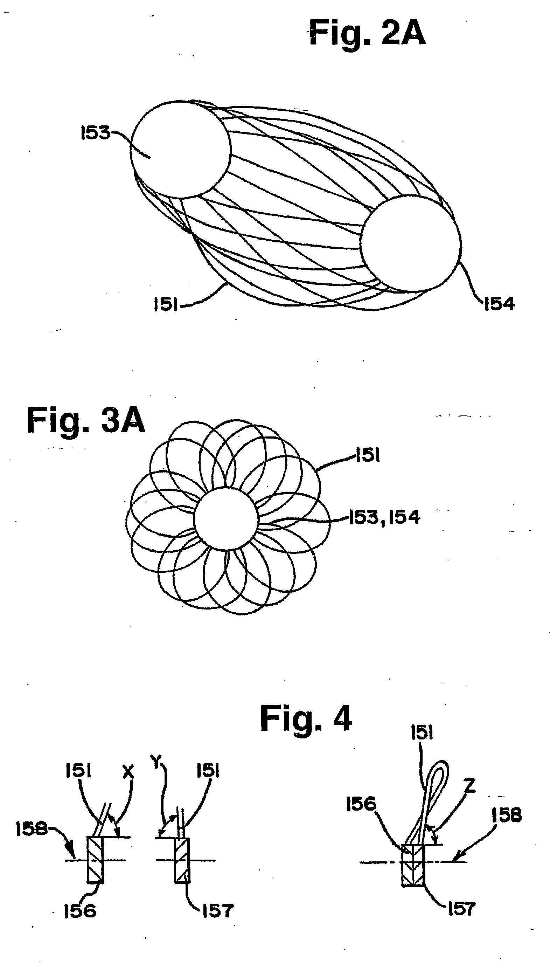

- FIG. 2A: shows the implant of FIG. 1 in a perspective view during extension;
- FIG. 3A: shows an end view of the implant of FIG. 1 in its extended position;
- FIG. 4: shows schematically the principle for building up the hub and connection of the implant of FIG. 1 to it and the final position of the implant in completely extended condition seen from the side;

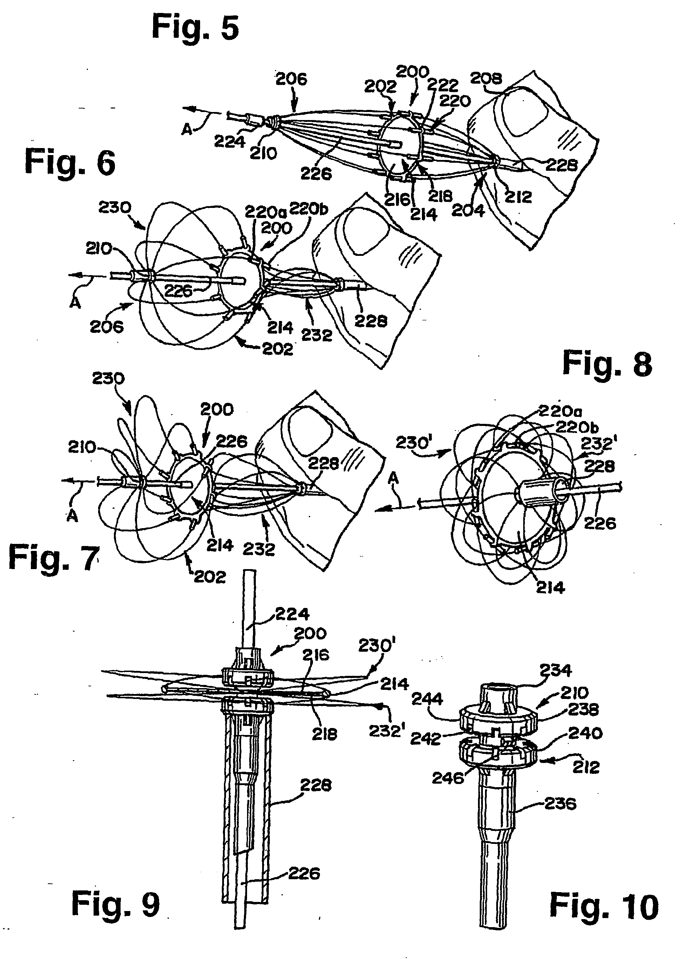

- FIG. 5: shows an embodiment of an implant according to the present invention in a perspective view in an initial position;
- FIG. 6: shows the implant of FIG. 5 in a perspective view in a first partially deployed position;
- FIG. 7: shows the implant of FIG. 5 in a perspective view in a second partially deployed position;
- FIG. 8: shows the implant of FIG. 5 in a perspective view in a fully deployed condition;
- FIG. 9: shows the implant of FIG. 5 in a side view in a fully deployed condition; and
- FIG. 10: shows an enlarged perspective view of a portion of the implant according to FIG. 5, without the membrane and loops, detailing the locking hubs.

### PREFERRED EMBODIMENTS

The device according to the invention is based on the implants disclosed in WO 02/38051.

This device or implant is employed as an implant for closing an internal passage, for example, an aperture in the auricle diaphragm or the ventricle diaphragm of a heart, or in a desired body channel which one wishes to close. A closing body is deployed or unfolded at the location. More particularly, the closing body is arranged to be expanded, preferably unfolded, at the position of the intended closing spot, after insertion through a body vein.

The closing body consists for example of an inflatable balloon or of an expansible, preferably unfoldable, disk-shaped member. The closing body is arranged to be dilated, expanded or unfolded radially by means of a number of stiffening means, which are made of non-bendable material and therefore twist when the distance between their fixed ends is reduced. A locking mechanism, preferably consisting of snap together members, are provided for locking the wire-like members in their twisted position.

This implant is delivered to the location for application in the form of a thin, elongated device and is then expanded or unfolded into its locking position.

FIGS. 1 - 4 disclose a device 101 not according to the invention being formed as an implant for closing a passage. This implant utilizes the rotating quality of non-bendable materials when they are compressed, and when the ends move past a critical point. For this object, there are thin threads attached around an inner core of material in each end inside a balloon. The distal and the proximal balloons have a further opening between them and the diameter of the balloon at its smallest part corresponding to the ASD-size, i.e. that it is somewhat but not much larger. The balloons are inflated by means of a contrast fluid whereupon the furthermost, as seen in the direction of insertion, attachment point of the threads are slowly pulled back at the same time as the nearest, as seen in the direction of insertion, are pushed ahead, i.e. in the direction toward each other. At a critical position, the threads twist or spiral sideways and maintain a circular shape, which desired shape is used according to the present invention. In position, the balloons are evacuated and the threads are locked in the twisted spring-like position.

The implant comprises a closing body 106 which is attachable at the passage, alone or in combination with further sealing means in a radial direction 107 which is also an expanding stiffening fluid tight closing body. The body 106 is arranged that after insertion through a body vein (not shown) in its longitudinal direction 150, it will be built up at the position 109 for the intended closing spot.

A number of threads 151 which in the shown embodiment are eight in number, are arranged of such a material and with such qualities that they twist automatically sideways to form a circular or loop shape when compressed longitudinally, as is shown in FIG. 3, and are locked in the twisted, spiral or coil-like position for interconnecting the two chambers, and so on, against each other. The body 106 is formed by a plurality of thin threads or wires, which are attached respectively in axial direction 150, 152 relative to each other on movable holder nuclei 153, 154. The threads 151 are arranged to twist sideways in the same direction and then to assume a somewhat circular shape 155, similar to a flower, a propeller or an umbrella, with circular shape and arranged with suitable means for closing the passage.

For example, the closing portion may be formed of layers of watertight material connected to the threads 151. These layers are preferably made of a biodegradable material as disclosed in the following example. Preferable, the whole implant is made of a biodegradable material.

In the example according to FIG. 4 the threads 151 are shown attached with different inclination x, y with reference to the respective separate holder nuclei 156, 157. The threads 151 are arranged to be mutually forced to be directed toward that holder nucleus 157 from which the threads 151 depart with the largest angle y, as calculated from the centre axis 158 of the holder nuclei, with similar angle z for the different threads 151.

FIGS. 5 - 10 further illustrate an embodiment of an implant according to the present invention:
FIG. 5 shows the implant 200 of the present invention in a perspective view in an initial position, i.e., before the device is applied to the passage in a circulatory system and deployed. In the figures a plurality of thin stiff generally inextensible, but somewhat flexible, members or wires 202 are provided. At least the members 202 are biodegradable. While the figure shows eight members, it should be understood that the number of members can be varied and not change the invention.

The members 202 are formed of a biodegradable or bioabsorbable material consisting of a polymer that exhibits a relatively high degree of biocompatibility. These materials have been previously used in stents, for example. Bioabsorbable implantable occlusive devices of the present invention are made completely or partly of polydioxanone, polycaprolactone or polygluconate, each of which have a characteristic degradation rate in the body. For example, PGA and polydioxanone are relatively fast-bioabsorbing materials (weeks to months) and PLA and polycaprolactone are relatively slow-bioabsorbing material (months to years). Reference is made to Enhancement of the Mechanical properties of polylactides by solid-state extrusion, W. Weiler and S. Gogolewski, Biomaterials 1996, Vo1..17 No. 5, pp. 529-535; and Deformation Characteristics of a Bioabsorbable Intravascular Stent, Investigative Radiology, December 1992, C. Mauli, Agrawal, Ph.D., P.E., H. G. Clark Ph.D., pp. 1020-1024.

Mechanical properties of these materials generally increase with increasing molecular weight. For instance, the strength and modulus of PLA generally increase with increasing molecular weight. Degradation time generally decreases with decreasing initial molecular weight (i.e., a mesh device made of a low molecular weight polymer would be bioabsorbed before a mesh device made of a high molecular weight polymer). Low molecular weight PLA is generally more susceptible to thermo-oxidative degradation than high molecular weight grades, so an optimum molecular weight range should be selected to balance properties, degradation time, and stability. The molecular weight and mechanical properties of the material generally decrease as degradation progresses. PLA generally has a degradation time greater than 1 year. Ethylene oxide sterilization process (EtO) is a preferred method of sterilization. Furthermore, PLA has a glass transition temperature of about 60 degrees C, so care must be taken not to expose products to high temperature environments for substantial time periods (temperatures greater than 60 degrees C), to avoid the possibility of dimensional distortion or other degradation effects.

PLA, PLLA, PDLA and PGA include tensile strengths of from about *275,79 N*/*mm² to about 827,37 N*/*mm²* (40 thousand of pounds per square inch (ksi) to about 120 ksi); a tensile strength of *5*5*1,58 N*/*mm²* (80 ksi) is typical; and a preferred tensile strength of from about *413,69 N*/*mm² to about 827,37 N*/*mm²* (60 ksi to about 120 ksi).

Polydioxanone, polycaprolactone, and polygluconate include tensile strengths of from about *103,42 N*/*mm² to about 413,69 N*/*mm²* (15 ksi to about 60 ksi); a tensile strength of about *241,32 N*/*mm²* (35 ksi) is typical; and a preferred tensile strength of from about 172,37 N/mm² to about 310,26 N/mm² (25 ksi to about 45 ksi).

PLA, PLLA, PDLA and PGA include tensile modulus of from about *2757,90 N*/*mm² to about 13789,52 N*/*mm²* (400,000 pounds per square inch (psi) to about 2,000,000) psi; a tensile modulus of *6205,28 N*/*mm²* (900,000 psi) is typical; and a preferred tensile modulus of from about *4826,33 N*/*mm² to about 8273,71 N*/*mm²* (700,000 psi to about 1,200,000 psi).

Polydioxanone, polycaprolactone, and polygluconate include tensile modulus of form about *1378,95 N*/*mm² to about 4826,33 N*/*mm²* (200,000 psi to about 700,000 psi); a tensile modulus of *3102,64 N*/*mm²* (450,000 psi) is typical; and a preferred tensile modulus of from about *2413,17 N*/*mm² to about 3792,12 N*/*mm²* (350,000 psi to about 550,000) psi.

PLLA filament has a much lower tensile strength. and tensile modulus than, for example, Elgiloy.RTM. metal alloy wire which may be used to make braided mesh devices or elements. The tensile strength of PLLA is about 22% of .the tensile strength of Elgiloy.RTM. The tensile modulus of PLLA is about 3% of the tensile modulus of Elgiloy.RTM. mechanical properties and self expansion are directly proportional to tensile modulus of the material. As a result, a PLLA filament or braided or woven element made to the same design as the implant of the present invention may have poor mechanical properties and may not be functional. The polymeric inextensible members 202 should have -radial strength similar to meta members and should have the required mechanical properties capable of bracing open endoluminal or the like strictures.

Each member 202 has a proximal end 204 and a distal end 206. The proximal end 204 is shown here positioned adjacent the user's hand 208 while the distal end 206 enters the passage (not shown) first. In practice, of course, the hand would typically be much further away, given that the device will be introduced intravenously. The distal ends 206 of the inextensible members 202 are attached to a first holder 210 that has a ring shape like that of a circular hub or hub flange. The proximal ends 204 of the inextensible members 202 are attached to a second holder 212 similar to the first holder 210, i.e., a ringshaped holder or hub.

At a point generally midway between the first holder 210 and the second holder 212 an expansible, preferably unfoldable, occluding body 214 is attached to the members 202. The occluding body 214 may be a generally circular, disc-shaped member, and may be made of a flexible fabric-like material consistent with surgical use and more preferably of a biodegradable material. The occluding body 214 has a distal face 216 oriented to-ward the distal ends 206 of the members 202 and a proximal face 218 oriented toward the proximal ends 204 of the members 202. The members 202 preferably have a thickened portion 220 where they pass through openings 222 in the occluding body 214. The thickened portion 220 is actually two parts 220a, 220b which serve to capture and mount the occluding body 214 therebetween.

The first holder 210 is attached to a first end 224 of a carrier rod 226 at the distal ends 206 of the members 202. The second holder 212 is slidably received on the carrier rod 226. In the condition shown in FIG. 11, the members 202 are arranged about the carrier rod 226 in a substantially equally spaced manner and generally aligned with the longitudinal axis A of the carrier rod 226. The occluding body 214 extends in a generally radial manner from the carrier rod 226 at a mid-point between the proximal 204 and distal ends 206 of the inextensible members. In the arrangement shown in FIG. 11, the implant 200 forms an elongated apparatus or article that generally extends along longitudinal axis A. The elongate form of the implant 200 permits it to be tightly compressed and inserted through an intravenous delivery mechanism and in the form shown is referred to as being in the insertion condition.

In operation, a driving implement 228, (e.g., a plastic tube) can be placed over the carrier rod 226 in contact with the second holder 212. The driving implement 228 is slid along or moved toward the first substantially fixed holder 210 (i.e., toward the first end 224 of the carrier rod 226) along the carrier rod 226. Generally, as will be further described, when the driving implement 228 is moved toward the first holder 210 the two holders 210, 212 are driven relatively closer together, and the implant 220 is changed into a deployed condition.

FIGS. 5 - 8 show the implant 200 in a perspective view in various conditions of deployment. As can be seen in these figures, moving the two holders 210, 212 together at first tends to cause the inextensible members 202 at the distal end to assume a gently outwardly bowed configuration with respect to the longitudinal axis A of the carrier rod 226. This outwardly bowed configuration pulls the expansible occluding body 214 into a tensioned radial disk shape.

Further moving the driving implement 228 towards the first holder 210 along the carrier rod 226 causes a first portion 230 of the inextensible members 202 located between the first holder 210 and the occluding body 214, to assume a more bowed, almost semicircular shape where the distal ends 206 of the members 202 attach to the first holder 210 in a nearly perpendicular direction with respect to the longitudinal axis A of the carrier rod 226. A second portion 232 of the inextensible members 202 at the proximal end tends to remain relatively parallel to the longitudinal axis A at first.

The inextensible members 202 are shown in FIG. 7 in a condition in response to yielding to further compression. As shown, the first portions 230 of the members 202 have started to twist or spiral about the carrier rod 226 forming a plurality of generally radial loops (with respect to the axis of the carrier rod). What then happens is that the first portion 230 then passes through a critical point, whereupon each wire in this portion 230 snaps into a generally radially-extending loop, yielding a somewhat concave petal-shaped structure at the distal side of the occluding body 214. Further compressing the second portion 232 likewise results in the formation of similar loops in a petal-shaped structure on the proximal side of the occluding body 214.

FIGS. 8 and 9 show the implant 200 in a perspective view in a fully deployed condition. Both the first portion 230 and second portion 232 have responded to the compressing motion by twisting away from the axial direction A. Thereby, the first portion 230 of the members have resolved into a first fixation structure 230' adjacent the distal face 216 of the occluding body. Similarly, the second portion 232 of the members 202 have resolved into a second fixation structure 232' adjacent the proximal face 218 of the occluding body 214. The overlapping spiral shape of both the first and second fixation structure 230', 232' is a stable shape, that locks the implant 200 into a stable occluding or deployed condition upon the locking holders 210, 212 being driven together by the driving implement 228, as shown in FIG. 11.

FIG. 10 shows a perspective view of a portion of the implant without the occluding body and members or carrier rod. Thus, the first and second holders or hubs 210, 212 may be seen in more detail. The first and second holders 210, 212 may be adapted to be placed onto the carrier rod (not shown) by hollow central shafts 234, 236. Located about the central shafts 234, 236 are annular portions 238, 240 with a plurality of slots 242 or attachment points sized to fixedly receive the ends of the members 202. The slots 242 may extend to the periphery 244 of the annular portions 238, 240 to allow the members 202 to move from a longitudinal orientation to a radial orientation.

An extending tooth 246 may be formed on one of the first and second holders 210, 212 or in the alternate, the carrier rod (not shown) to engage and form a locking element with a corresponding recess (not shown) formed in one or both of the first and second holders 210, 212. In this manner, the implant 200 becomes locked into a fully deployed or final occluding condition upon being driven together by the driving implement by engagement of the tooth 246 and the recess.

The invention has been carefully described in the above-mentioned examples, and therefore the idea should be clearly understood that the invention is neither limited to the above described and on the drawings shown embodiment, but may be varied within the scope of the claims without departing from the concept of the invention.

## Claims

1. An implant for occluding a passage in a circulatory system, comprising:
a plurality of thin, preferably stiff, generally inextensible members (202) each having a proximal and a distal end;
a first holder (210) to which the distal ends of said members are attached;
a second holder (212) to which the proximal ends of said members are attached;
an expansible, preferably foldable, occluding body (214) attached to said members (202);
said plurality of members (202) with said occluding body (214) forming an elongated article extending along a longitudinal axis (A) and being adapted for insertion through a delivery mechanism in an insertion condition;
said members (202) being attached to said first and second holders (210, 212) and the distance between said holders being reduceable past a critical point wherein said members (202) twist sideways as a response to this reduction of the distance past the critical point, the twisting motion being relative to said axis to yield a plurality of generally radially extending loops forming at least one fixation structure (230', 232'), thereby expanding, preferably unfolding, the occluding body (214) and said at least one fixation structure (230'; 232') being fixable in a final occluding condition,
**characterized in that** said plurality of members (202) are *formed completely* of biodegradable material, which consists of polydioxanone, polycaprolactone or polygluconate comprising a tensile strength of from about *103,42 N*/*mm² to about 413,69 N*/*mm²* (15 thousands of ponds per square inch (ksi) to about 60 ksi) and / or comprising a tensile modulus of from about *1378,95 N*/*mm² to about 4826,33 N*/*mm²* (200,000 pounds per square inch (psi) to about 700,000 psi).

2. The implant of claim 1, wherein the occluding body (214) is a generally disc-shaped member.

3. The implant according to one of claims 1 or 2, wherein said occluding body (214) is arranged at a point intermediate said first and second holders (210, 212), said occluding body (214) having a distal and proximal face (216, 218).

4. The implant according to one of claims 1 to 3, wherein said radially extending loops form a first fixation structure (230') adjacent said distal face of said occluding body (214) and a second fixation structure (232') adjacent said proximal face of said occluding body (214).

5. The implant according to one of the preceding claims, wherein the biodegradable material consists of polydioxanone, polycaprolactone or polygluconate comprising a preferred tensile strength of from about *172,37 N*/*mm² to about 310,26 N*/*mm²* (25 ksi to about 45 ksi).

6. The implant according to one of the preceding claims, wherein the biodegradable material consists of polydioxanone, polycaprolactone or polygluconate comprising a preferred tensile modulus of from about 2*41,32 N*/*mm² to about 379,21 N*/*mm²* 350,000 psi to about 550,000 psi).

7. The implant according to one of the preceding claims, wherein said twisting motion causes said members (202) attached to said first holder (230') to initially bulge radially outwardly and then snap into an inverted condition having a concave shape for said radially extending loops of said first fixation structure (230'), with the concavity facing said first holder (210).

8. The implant according to one of the preceding claims, wherein said members are generally inextensible and have a thickened portion where said members attach to said expansible occluding body.

9. The implant according to one of the preceding claims, wherein each said first and second holders (210, 212) include a hollow central shaft portion (234, 236) and an annular hub portion (238, 240) positioned about the central shaft portion.

10. The implant of claim 9, wherein each said annular hub portion (238, 240) includes a plurality of slots (242) adapted to respectively receive one of said proximal and distal ends of said members (202).

11. The implant of claim 10, wherein each of said slots (242) extend to an outer periphery of said annular hub portions (238, 240) of said first and second holders (210, 212) to permit said inextensible members (202) to pivot from a first orientation generally parallel to said longitudinal axis (A) to a second orientation generally radial with respect to said longitudinal axis.

## Patentansprüche

1. Ein Implantat zum Verschliessen eines Durchgangs in einem Kreislaufsystem, welches umfasst:
eine Vielzahl von dünnen, vorzugsweise steifen, im Wesentlichen undehnbaren Gliedern (202) mit jeweils einem proximalen und einem distalen Ende;
einen ersten Halter (210), an welchem die distalen Enden dieser Glieder angebracht sind;
einen zweiten Halter (212), an welchem die proximalen Enden dieser Glieder angebracht sind;
einen expandierbaren, vorzugsweise faltbaren Verschlusskörper (214), der an diesen Gliedern (202) angebracht ist;
wobei in einem Einführzustand diese Vielzahl von Gliedern (202) mit diesem Verschlusskörper (214) einen langgestreckten Gegenstand bilden, der sich entlang einer longitudinalen Achse (A) erstreckt und zum Einführen durch einen Zuführmechanismus geeignet ist;
wobei diese Glieder (202) an diesen ersten und zweiten Haltern (210; 212) angebracht sind, und der Abstand zwischen diesen Haltern über einen kritischen Punkt hinaus verkleinerbar ist, wobei die Glieder (202) eine seitliche Twistbewegung durchführen als Antwort auf diese Verkleinerung des Abstandes über den kritischen Punkt hinaus, wobei die Twistbewegung relativ zu dieser Achse geschieht, um eine Vielzahl von sich hauptsächlich radial erstreckenden Schlaufen zu bilden, die zumindest eine Fixierungsstruktur (230'; 232') formen und dabei den Verschlusskörper (214) expandieren, vorzugsweise auffalten, und wobei diese zumindest eine Fixierungsstruktur (230'; 232') in einem endgültigen Verschlusszustand fixierbar ist,
**dadurch gekennzeichnet, dass** diese Vielzahl von Gliedern (202) vollständig aus biologisch abbaubarem Material ausgebildet sind, welches aus Polydioxanon, Polycaprolacton oder Polygluconat besteht, das eine Zugfestigkeit von ca. 103.42 N/mm² bis ca. 413.69 N/mm² (15 Tausende von Pfunden pro Quadratzoll (ksi) bis ca. 60 ksi) aufweist und / oder das einen Zugmodul von ca. 1378.95 N/mm² bis ca. 4826.33 N/mm² (200'000 Pfunde pro Quadratzoll (psi) bis ca. 700'000 psi) hat.

2. Das Implantat nach Anspruch 1, wobei der Verschlusskörper (214) ein im Wesentlichen plattenförmiges Glied ist,

3. Das Implantat nach einem der Ansprüche 1 oder 2, wobei der Verschlusskörper (214) an einem Punkt zwischen den ersten und zweiten Haltern (210, 212) angeordnet ist, und wobei der Verschlusskörper (214) eine distale und eine proximale Fläche (216, 218) aufweist.

4. Das Implantat nach einem der Ansprüche 1 bis 3, wobei die sich radial erstreckenden Schlaufen eine erste Fixierungsshuktar (230') angrenzend an die distale Fläche des Verschlusskörpers (214) bilden und eine zweite Fixierungsstruktur (232') angrenzend an die proximale Fläche des Verschlusskörpers (214).

5. Das Implantat nach einem der vorangehenden Ansprüche, wobei das biologisch abbaubare Material aus Polydioxanon, Polycaprolacton oder Polygluconat besteht, das eine bevorzugte Zugfestigkeit von ca. 172.37 N/mm² bis ca. 310.26 N/mm² (25 ksi bis ca. 45 ksi) aufweist.

6. Das Implantat nach einem der vorangehenden Ansprüche, wobei das biologisch abbaubare Material aus Polydioxanon, Polycaprolacton oder Polygluconat besteht, das einen bevorzugten Zugmodul von ca. 241.32 N/mm² bis ca. 379.21 N/mm² (350'000 psi bis ca. 550'000 psi) aufweist.

7. Das Implantat nach einem vorangehenden Ansprüche, wobei die Twistbewegung die an dem ersten Halter (210) angebrachten Gliedern (202) veranlasst, sich anfänglich radial nach aussen zu wölben und dann in einen umgekehrten Zustand umzuschnappen, der eine konkave Form für die sich radial erstreckenden Schlaufen der ersten Fixierungsstruktur (230') aufweist, wobei die Wölbung zum ersten Halter (210) ausgerichtet ist

8. Das Implantat nach einem vorangehenden Ansprüche, wobei diese Glieder im Wesentlichen nicht dehnbar sind und einen verdickten Bereich haben, an dem die Glieder an dem expandierbaren Verschlusskörper angebracht sind.

9. Das Implantat nach einem vorangehenden Ansprüche, wobei der erste und der zweite Halter (210, 212) jeweils einen hohlen zentralen Achsenbereich (234, 236) und einen ringförmigen Buchsenbereich (238, 240), der an dem zentralen Achsenbereich angeordnet ist, umfassen.

10. Das Implantat nach Anspruch 9, wobei jeder der ringförmigen Buchsenbereiche (238, 240) eine Vielzahl von Schlitzen (242) aufweist, die zur Aufnahme jeweils eines der proximalen und distalen Enden der Glieder (202) geeignet sind.

11. Das Implantat nach Anspruch 10, wobei sich jeder der Schlitze (242) bis zu einem Aussenumfang des ringfönnigen Buchsenbereichs (238, 240) des ersten und zweiten Halters (210, 212) erstreckt, um den nicht dehnbaren Gliedern (202) ein Verschwenken von einer ersten im wesentlichen parallel zur longitudinalen Achse (A) verlaufenden Orientierung zu einer zweiten im wesentlichen radial in Bezug zu dieser longitudinalen Achse verlaufenden Orientierung zu ermöglichen.

## Revendications

1. Implant pour occlure un passage dans un système circulatoire, comprenant :
une pluralité d'éléments (202) minces, de préférence rigides, généralement inextensibles, chacun comportant une extrémité proximale et une extrémité distale ;
un premier support (210) auquel les extrémités distales desdits éléments sont attachées ;
un second support (212) auquel les extrémités proximales desdits éléments sont attachées ;
un corps occlusif (214) dilatable, de préférence pliable, attaché auxdits éléments (202) ;
ladite pluralité d'éléments (202) avec ledit corps occlusif (214) formant un article allongé s'étendant le long d'un axe longitudinal (A) et étant adapté pour une insertion à travers un mécanisme de délivrance dans un état d'insertion ;
lesdits éléments (202) étant attachés auxdits premier et second supports (210, 212) et la distance entre lesdits supports étant réductible après un point critique au-delà duquel lesdits éléments (202) se tordent latéralement en réponse à cette réduction de la distance après le point critique; le mouvement de torsion étant relatif audit axe pour produire une pluralité de boucles s'étendant généralement radialement formant au moins une structure de fixation (230', 232'), dilatant ainsi, de préférence dépliant le corps occlusif (214), et ladite au moins une structure de fixation (230' ; 232') pouvant être fixée dans un état occlusif final,
**caractérisé en ce que** ladite pluralité d'éléments (202) est formée entièrement en matériau biodégradable, qui consiste en une polydioxanone, une polycaprolactone ou un polygluconate présentant une résistance à la traction d'environ 103,42 N/mm² à environ 413,69 N/mm² (15 milliers de livres par pouce carré (ksi) à environ 60 ksi) et/ou présentant un module d'élasticité en traction d'environ 1378,95 N/mm² à environ 4826,33 N/mm² (200 000 livres par pouce carré (psi) à environ 700 000 psi).

2. Implant selon la revendication 1, dans lequel le corps occlusif (214) est un élément généralement en forme de disque.

3. Implant selon l'une des revendications 1 et 2, dans lequel ledit corps occlusif (214) est disposé en un point intermédiaire entre lesdits premier et second supports (210, 212), ledit corps occlusif (214) comportant une face distale et une face proximale (216, 218).

4. Implant selon l'une des revendications 1 à 3, dans lequel lesdites boucles s'étendant radialement forment une première structure de fixation (230') adjacente à ladite face distale dudit corps occlusif (214) et une seconde structure de fixation (232') adjacente à ladite face proximale dudit corps occlusif (214).

5. Implant selon l'une des revendications précédentes, dans lequel le matériau biodégradable consiste en une polydioxanone, une polycaprolactone ou un polygluconate présentant une résistance à la traction préférée d'environ 172,37 N/mm² à environ 310,26 N/mm² (25 ksi à environ 45 ksi).

6. Implant selon l'une des revendications précédentes, dans lequel le matériau biodégradable consiste en une polydioxanone, une polycaprolactone ou un polygluconate présentant un module d'élasticité en traction préféré d'environ 241,32 N/mm² à environ 379,21 N/mm² (350 000 psi à environ 550 000 psi).

7. Implant selon l'une des revendications précédentes, dans lequel ledit mouvement de torsion force lesdits éléments (202) attachés audit premier support (230') d'abord à se renfler radialement vers l'extérieur puis à se refermer dans un état inversé ayant une forme concave pour lesdites boucles s'étendant radialement de ladite première structure de fixation (230'), avec la concavité faisant face audit premier support (210).

8. Implant selon l'une des revendications précédentes, dans lequel lesdits éléments sont généralement inextensibles et ont une partie épaissie où lesdits éléments se fixent audit corps occlusif dilatable.

9. Implant selon l'une des revendications précédentes, dans lequel chacun desdits premier et second supports (210, 212) comprend une partie d'axe central creuse (234, 236) et une partie de moyeu annulaire (238, 240) positionnée autour de la partie d'axe central.

10. Implant selon la revendication 9, dans lequel chacune desdites parties de moyeu annulaire (238, 240) comprend une pluralité de fentes (242) adaptées pour recevoir respectivement l'une desdites extrémités proximales et distales desdits éléments (202).

11. Implant selon la revendication 10, dans lequel chacune desdites fentes (242) s'étend jusqu'à une périphérie externe desdites parties de moyeu annulaire (238, 240) desdits premier et second supports (210, 212) pour permettre auxdits éléments inextensibles (202) de pivoter depuis une première orientation généralement parallèle audit axe longitudinal (A) jusqu'à une seconde orientation généralement radiale par rapport audit axe longitudinal.
